# EUROPÄISCHE PATENTSCHRIFT

(11) **EP 0 511 986 B1**
(45) Veröffentlichungstag und Bekanntmachung des Hinweises auf die Patenterteilung: **14.12.1994**
(21) Anmeldenummer: 91902198.0
(22) Anmeldetag: 15.01.1991
(51) Int. Cl.: C07C 69/587, C07C 67/347

(54) **OLEFINISCH UNGESÄTTIGTE ADDUKTE VON ETHYLEN AN MEHRFACH UNGESÄTTIGTE FETTSÄUREN BZW. FETTSÄUREESTER UND VERFAHREN ZU IHRER HERSTELLUNG**
OLEFINICALLY UNSATURATED ADDUCTS OF ETHYLENE ON POLYUNSATURATED FATTY ACIDS OR FATTY ACID ESTERS AND PROCESS FOR PRODUCING THEM
PRODUITS D'ADDITION OLEFINIQUEMENT INSATURES DE L'ETHYLENE SUR DES ACIDES GRAS MULTI-INSATURES OU SUR LEURS ESTERS ET LEUR PROCEDE DE FABRICATION

(30) Priorität: 24.01.1990 DE 4002012
(43) Veröffentlichungstag der Anmeldung: 11.11.1992
(73) Patentinhaber: Henkel Kommanditgesellschaft auf Aktien, 40191 Düsseldorf (DE)
(72) Erfinder: LAUFENBERG, Alfred, Dr., D-4047 Dormagen 5 (DE); BEHR, Arno, Dr., D-4000 Düsseldorf 13 (DE)
(86) Internationale Anmeldenummer: EP9100054
(87) Internationale Veröffentlichungsnummer: WO9111428

(56) Entgegenhaltungen:
- EP-A- 0 206 367
- US-A- 3 966 798
- US-A- 4 318 860

## Beschreibung

Die Erfindung betrifft olefinisch ungesättigte Addukte von Ethylen an mehrfach ungesättigte Fettsäuren mit 18 bis 22 Kohlenstoffatomen bzw. an Ester derselben mit C₁-C₃₆-Alkanolen in molaren Verhältnissen von Ethylen zu den Fettsäuren bzw. Fettsäureestern im Bereich von 1 : 1 bis 3 : 1, erhältlich durch Umsetzung der Fettsäuren bzw. Fettsäureester mit Ethylen bei erhöhter Temperatur und erhöhtem Druck in Gegenwart von Verbindungen von Übergangsmetallen aus der von Ru, Rh, Pd, Ir und Pt gebildeten Gruppe als Katalysatoren.

In der Alkylkette verzweigte Fettsäuren vom Typ der Guerbetsäuren, erhältlich durch Guerbetisierung der entsprechenden Fettalkohole und Oxidation der Guerbetalkohole zu den entsprechenden Säuren, sind technologisch interessante Zwischenprodukte, weil sie bzw. ihre Alkylester, im Vergleich zu den entsprechenden unverzweigten Isomeren, deutlich erniedrigte Stockpunkte aufweisen. Die Herstellung von Guerbetsäuren ist jedoch technologisch aufwendig und nur mit unbefriedigenden Ausbeuten durchführbar. Es hat daher nicht an Versuchen gefehlt, ausgehend von Fettsäuren bzw. Estern derselben entsprechende Fettsäurederivate herzustellen, die in der Alkylkette Verzweigungen aufweisen. Ein typisches Beispiel hierfür ist die schichtsilikatkatalysierte Fettsäuredimerisation, bei der jedoch auch erhebliche Mengen an trimeren Fettsäuren sowie an methylverzweigten Fettsäuren, sogenannten Isofettsäuren, gebildet werden. Ein weiteres, wenn auch aufwendiges Verfahren liefert aus Konjuenfettsäuren in der trans,trans-Form mit aktivierten Dienophilen unter den Bedingungen einer Diels-Alder-Reaktion verzweigte Fettsäurederivate; so läßt sich z.B. auf diesem Wege aus Linolsäure und Acrylsäure eine verzweigte C₂₁-Dicarbonsäure herstellen, vgl. US-B 3,734,859, US-B 3,753,968, DE-B 2 253 930.

Des weiteren sind aus der EP-A1 0 010 807 verzweigte Monocarbonsäuren bekannt, die durch Telomerisation von einem Mol Acetanhydrid und mindestens drei Mol eines Olefins mit mindestens 6 und bis zu 30 Kohlenstoffatomen in Gegenwart einer dreiwertigen Manganverbindung erhalten werden.

Weitere, verzweigte Fettsäurederivate sind durch thermische oder säurekatalysierte Addition von aktivierten Enophilen an ungesättigte Fettsäurederivate erhalten worden. So läßt sich z.B. Maleinsäureanhydrid unter Säurekatalyse mit bis zu 70 % Ausbeute an Ölsäure addieren, vgl. Fat. Sci. Technol., 1, 1 (1988). Bei den vorstehend genannten Reaktionsprodukten erweist sich die Anwesenheit von mehr als einer Carboxylgruppe jedoch häufig als störend.

Schließlich hat man auch versucht, gesättigte Kohlenwasserstoffe durch thermisch initiierte radikalische Addition von gesättigten Kohlenwasserstoffen an Fettsäuren anzulagern; die Addition von Cyclohexan an Ölsäuremethylester führt bei 340°C und 200 bar mit 70%-iger Selektivität, jedoch mit einer Ausbeute von lediglich 2,8 %, zu alkylverzweigten Fettsäuren, vgl. J.O. Metzger, et al., Fat. Sci. Technol. 1 (1989), 18.

Die Erfindung ist auf die Bereitstellung olefinisch ungesättigter Addukte von Ethylen an mehrfach ungesättigte Fettsäuren der eingangs genannten Art gerichtet, die auf einfache Weise und mit guten Ausbeuten hergestellt werden können. Die erfindungsgemäß bereitgestellten Verbindungen sind neue Produkte, die sich z.B. von in der Natur vorkommenden ethylverzweigten Fettsäuren mit insgesamt 12 bis 18 Kohlenstoffatomen, beschrieben in A. Smith et al, Biomed. Mass Spectrom., 6 (8), 347-349, bereits durch ihre Kettenlänge unterscheiden.

Als Ausgangsprodukte zur Herstellung der olefinisch ungesättigten Addukte der Erfindung eignen sich ungesättigte Fettsäuren mit 18 bis 22 Kohlenstoffatomen und mehr als einer olefinischen Doppelbindung wie Linolsäure, isomerisierte Linolsäure mit konjuierten Doppelbindungen (sogenannte C18:2-Konjuenfettsäure), Linolensäure, Arachidonsäure, Docosadiensäure, Docosahexaensäure und Eicosapentaensäure, die in Form ihrer technischen Gemische mit anderen Fettsäuren nachwachsenden natürlichen Rohstoffen, z.B. aus Sonnenblumenöl, Tallöl oder Fischöl, erhalten werden können. Diese mehrfach ungesättigten Fettsäuren werden, wie in der Fettchemie üblich, im allgemeinen nicht in Form ihrer reinen Verbindungen, sondern in Form ihrer technischen Gemische zur Herstellung der Addukte der Erfindung eingesetzt. Die vorgenannten Fettsäuren werden bevorzugt nicht nur als solche, sondern auch in Form ihrer Ester mit C₁-C₃₆-Alkanolen, insbesondere mit C₁-C₄-Alkanolen, eingesetzt. Typische Beispiele für derartige Alkanole zur Bildung von Estern mit den vorgenannten Fettsäuren sind Methanol, Ethanol, Propanol, Butanol, Pentanol, Hexanol, Octanol, Decanol, Dodecanol, Tetradecanol, Hexadecanol, Octadecanol sowie höhere Fettalkohole bzw. Fettalkoholderivate mit bis zu 36 Kohlenstoffatomen, z.B. C₃₆-Guerbetalkohole.

Die vorgenannten mehrfach ungesättigten Fettsäuren bzw. Fettsäureester lagern erfindungsgemäß bei erhöhter Temperatur und erhöhtem Druck in Gegenwart von Verbindungen von Übergangsmetallen aus der von Ru, Rh, Pd, Ir und Pt gebildeten Gruppe Ethylen an.

Typische Beispiele für erfindungsgemäß einsetzbare Katalysatoren sind die folgenden:
RhCl₃.3H₂O
RhBr₃.3H₂O
[(C₂H₄)₂RhCl]₂
Rh(NO₃)₃.2H₂O
Rh(OOCCH₃)₂.2H₂O
Rh(acetylacetonat)₃
RhF₃.6H₂O
RhJ₃
Rh(CN)₃.3H₂O
Rh₂(SO₄)₃
Rh₂(CO₃)₃
[(1.5-Cyclooctadien)RhCl]₂
[(C₂H₄)₂Rh(acetylacetonat)]
[(1.3-Butadien)RhCl]₂
Cyclopentadienyl-Olefin-Komplexe wie [(n-C₅H₅)Rh(C₂H₄)₂].

Sofern die erfindungsgemäß einsetzbaren Katalysatoren in wasserfreier Form vorliegen, kann es zweckmäßig sein, dem Reaktionsgemisch eine geringe Menge von Wasser zuzusetzen.

Die im Rahmen der Erfindung einsetzbaren Katalysatoren sind als solche für die Anlagerung von Ethylen an Alkadiene bekannt, vgl. US-B 3,636,122; M. Bochmann et al., Journal of Molecular Catalysis, 22 (1984) 363-365; G. Wilkinson (Ed.), Comprehensive Organometallic Chemistry, Seite 414-429, Pergamon Press (1982); A.C.L. Su, Advances in Organometallic Chemistry, Vol. 17, Seite 271-283; wobei diese Veröffentlichungen, auf deren Inhalt im übrigen ausdrücklich Bezug genommen wird, sich jedoch nicht mit der Anlagerung von Alkenen an Fettsäuren bzw. Fettsäurederivate oder andere Fettstoffe befassen.

Weitere, im Verfahren der Erfindung einsetzbare Katalysatoren sind z.B.
PdCl₂
PtCl₂
IrCl₃
Ru(acetylacetonat)₃.

Mit den erfindungsgemäß einsetzbaren Katalysatoren entstehen im allgemeinen Gemische von 1:1-, 2:1- und 3:1-Addukten von Ethylen an die Fettsäuren bzw. Fettsäureester. Durch Veränderung der Reaktionsbedingungen wie Druck, Temperatur und Reaktionszeit lassen sich jedoch die Anteile an den verschiedenen Addukten variieren. Setzt man dem Reaktionsgemisch jedoch neben den Katalysatoren geeignete Phosphin- oder Phosphitliganden, z.B.
P(C₄H₉)₃
P(OC₄H₉)₃
P(C₆H₅)₃
P(OC₆H₅)₃
oder andere, aus dem zuletzt diskutierten Stand der Technik sowie der DE-B 20 16 133 bekannte Liganden zu, kann man unter Umständen gezielt die Zusammensetzung der Adduktgemische beeinflussen; so werden bei Reaktionssystemen, die ohne derartige Liganden überwiegend 2:1-Addukte liefern, beim Einsatz großer Liganden überwiegend 3:1-Addukte und beim Einsatz kleiner Liganden überwiegend 1:1-Addukte gebildet. Ähnliche Effekte lassen sich zum Teil dadurch erreichen, daß man dem Reaktionssystem Promotoren wie LiCl, FeCl₃ oder AgBF₄ zusetzt, die als solche ebenfalls aus dem zuletzt genannten Stand der Technik bekannt sind.

Die Struktur der erfindungsgemäßen olefinisch ungesättigten Addukte ist nicht einheitlich. Im Falle der Linolsäure (bzw. der von dieser abgeleiteten C₁₈-Konjuenfettsäure) konnte gezeigt werden, daß die Anlagerung des ersten Ethylenmoleküls zwischen den Positionen 9 und 12 der Kohlenstoffkette der Linolsäure erfolgt, wobei das 1:1-Addukt die gleiche Anzahl von Doppelbindungen wie die als Ausgangsmaterial eingesetzte Fettsäure aufweist. Die Stellung der Doppelbindungen ist jedoch unsicher. Die Doppelbindungen sind in keinem Fall mehr als 4 Kohlenstoffatome von der Verzweigung entfernt und stehen grundsätzlich in alpha,delta- oder in alpha,gamma-Stellung zueinander. Das zweite und gegebenenfalls das dritte Ethylenmolekül wird dann an eine in der Verzweigung befindliche Doppelbindung angelagert. Es ist zu vermuten, daß die erfindungsgemäß erhaltenen Addukte mindestens teilweise eine der auf der folgenden Seite wiedergegebenen Strukturen aufweisen.

Gemäß einer vorteilhaften Ausführungsform der Erfindung weisen die gegebenenfalls in Form ihrer Ester eingesetzten mehrfach ungesättigten Fettsäuren zwei bis 5, insbesondere 2 bis 3 olefinische Doppelbindungen auf.

Gemäß einer weiteren vorteilhaften Ausführungsform der Erfindung erhält man die Addukte bei einem Ethylendruck im Bereich von 5 bis 60 bar und einer Temperatur im Bereich von 50 bis 140°C, wobei man gegebenenfalls in Anwesenheit inerter organischer Lösemittel wie Hexan, Chloroform oder dergleichen, arbeitet.

Gemäß einer weiteren vorteilhaften Ausführungsform der Erfindung verwendet man die Katalysatoren in einer Menge im Bereich von 0,02 bis 2 Mol-%, bezogen auf Fettsäuren bzw. Fettsäureester.

Vorteilhafterweise verwendet man als Katalysatoren Rhodiumverbindungen, wobei wiederum Rhodiumverbindungen aus der von RhCl₃ und RhBr₃ (einschließlich Hydraten derselben) sowie [(C₂H₄)₂RhCl]₂ gebildeten Gruppe als Katalysatoren bevorzugt sind. 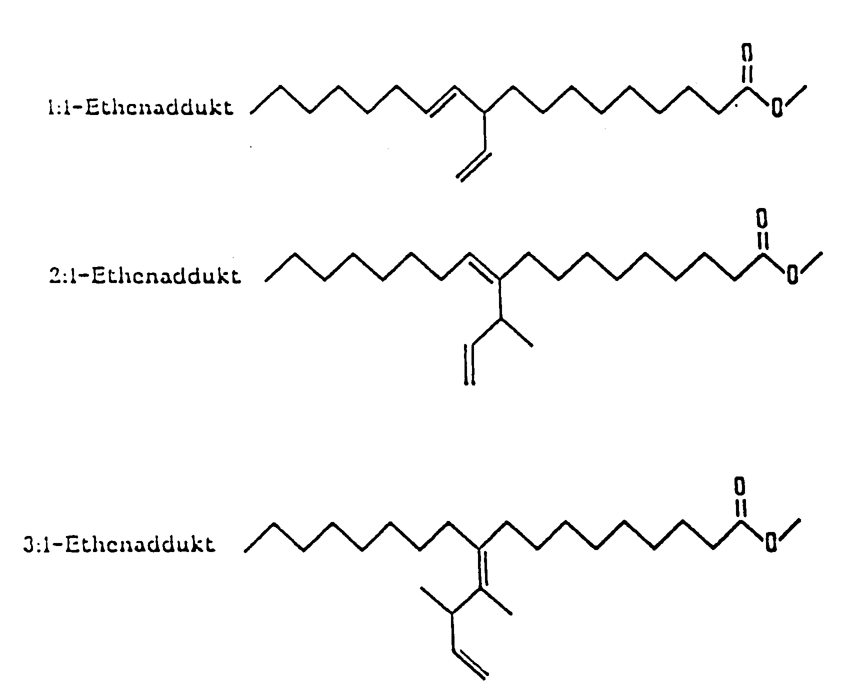
Die Erfindung betrifft weiterhin ein Verfahren zur Herstellung von olefinisch ungesättigten Addukten von Ethylen an mehrfach ungesättigte Fettsäuren mit 18 bis 22 Kohlenstoffatomen bzw. an Ester derselben mit C₁-C₃₆-Alkanolen in molaren Verhältnissen von Ethylen zu den Fettsäuren bzw. Fettsäureestern im Bereich von 1:1 bis 3:1 mit den oben erläuterten Merkmalen.

Die olefinisch ungesättigten Addukte der Erfindung sind als Ausgangsprodukte für die Herstellung gesättigter, verzweigter Fettsäuren mit 20 bis 28 Kohlenstoffatomen bzw. Ester derselben mit C₁-C₃₆-Alkanolen, die z.B. als Schmiermitteladditive oder in kosmetischen Formulierungen eingesetzt werden können, geeignet.

Die Erfindung wird im folgenden anhand bevorzugter Ausführungsbeispiele näher erläutert.

### Beispiel 1.

8,2 g eines technischen Fettsäuregemisches mit einem Anteil von 56 Gew.-% konjugierter C18:2-Fettsäure wurden mit 100 mg RhCl₃.3H₂O in 10 ml Hexan 20 h bei 100°C und 30 bar Ethylenkaltdruck in einem 75 ml-Stahlautoklaven umgesetzt. Man erhielt (gaschromatografisch ermittelt) 63,1 % Ethylenaddukte, bezogen auf Konjuenfettsäure, davon 34,9 % 1:1-, 44,2 % 2:1- und 20,9 % 3:1-Addukte.

### Beispiel 2.

2,5 kg einer technischen Konjuenfettsäure gemäß Beispiel 1, 0,8 g RhCl₃.3H₂O und 1 l Hexan wurden 20 h bei 100°C und 20 bar konstantem Ethylendruck in einem 5 l-Rührautoklaven mit Turbinenrührer umgesetzt. Die Ausbeute an Ethylenaddukten betrug 89,4 %, davon 23,7 % 1:1-, 41,7 % 2:1- und 34,6 % 3:1-Addukte.

### Beispiel 3.

8,2 g eines technischen Linolsäuremethylesters (67,8 % Methyllinolat, 22,4 % Methyloleat) und 100 mg RhCl₃.3H₂O wurden mit 10 ml Chloroform bei 100°C und einem Ethylenkaltdruck von 20 bar umgesetzt. Die Adduktausbeute, bezogen auf Methyllinolat, betrug 57,8 %, davon 30,8 % 1:1-, 42,0 % 2:1- und 27,2 % 3:1-Addukte.

### Beispiel 4.

Die Wiederholung des Beispiels 3 bei einem Ethylenkaltdruck von 30 bar ergab eine Adduktausbeute von 57,6 % bei Anteilen von 31,5 % 1:1-, 52,2 % 2:1- und 16,3 % 3:1-Addukten.

### Beispiel 5.

8,2 g eines technischen C18:2-Konjuenmethylesters (enthaltend 60,3 % Konjuenester, 6,2 % Linolsäuremethylester und 24,4 % Ölsäuremethylester) in 10 ml Chloroform wurden bei 100°C und 20 bar Ethylenkaltdruck umgesetzt. Man erhielt 93,5 % Ethylenaddukte, davon 27,5 % 1:1-, 51,1 % 2:1- und 21,4 % 3:1-Addukte.

### Beispiel 6.

Die Wiederholung des Beispiels 5 bei einem Ethylenkaltdruck von 30 bar ergab eine Gesamtausbeute an Ethylenaddukten von 88,5 %, davon 36,5 % 1:1-, 51,0 % 2:1- und 12,1 % 3:1-Addukte.

### Beispiel 7.

Die Wiederholung des Beispiels 6 unter Zusatz von 185,1 mg FeCl₃ als Promotor ergab eines Gesamtadduktausbeute von 81,8 %, davon 22,8 % 1:1-, 46,9 % 2:1- und 30,3 % 3:1-Addukte.

### Beispiel 8.

Die Wiederholung des Beispiels 6 mit Hexan anstelle von Chloroform ergab eine Gesamtadduktausbeute von 87,4 %, davon 24,8 % 1:1-, 59,7 % 2:1- und 15,5 % 3:1-Addukte.

### Beispiel 9.

Die Wiederholung des Beispiels 6 mit 74 mg [(C₂H₄)₂RhCl]₂ anstelle von RhCl₃.3H₂O ergab eine Gesamtadduktausbeute von 78,0 %, davon 24,2 % 1:1-, 61,8 % 2:1- und 13,0 % 3:1-Addukte.
Die Gesamtausbeute stieg auf 86,5 %, wenn man dem Reaktionsgemisch 86,4 mg P(C₄H₉)₃ zusetzte.

### Beispiel 10.

Die Wiederholung des Beispiels 6 unter Verwendung von 13 mg RhBr₃.3H₂O anstelle von RhCl₃.3H₂O sowie in Abwesenheit eines Lösemittels ergab eine Gesamtausbeute an Ethylenaddukten, bezogen auf Konjuenestergehalt, von 94,6 %, davon 17,7 % 1:1-, 62,8 % 2:1- und 16,5 % 3:1-Addukte.

### Beispiel 11.

Einfluß von Phosphin- und Phosphitliganden.

Das Beispiel 3 wurde unter Zusatz von 76,76 (0,380 mmol) P(C₄H₉)₃ wiederholt. Die Gesamtausbeute an Addukt betrug 54,5 %, bezogen auf Methyllinolat, davon 32,4 % 1:1-, 16,2 % 2:1- und 6,0 % 3:1-Addukt.

Die Wiederholung mit 9,50 mg (0,038 mmol) P(OC₄H₉)₃ ergab eine Gesamtadduktausbeute von 57,8 %, davon 34,9 % 1:1-, 16,4 % 2:1- und 6,5 % 3:1-Addukt.

Die Wiederholung mit 9,96 mg (0,038 mmol) P(C₆H₅)₃ ergab eine Gesamtadduktausbeute von 64,2 %, davon 8,7 % 1:1-, 17,9 % 2:1- und 37,5 % 3:1-Addukt.

Die Wiederholung mit 117,8 mg (0,38 mmol) P(OC₆H₅)₃ ergab eine Gesamtadduktausbeute von 59,2 % bei ähnlicher Adduktverteilung wie bei P(C₆H₅)₃.

### Beispiel 12.

Das Beispiel 8 wurde mit verschiedenen Katalysatoren anstelle des dort beschriebenen Katalysators wiederholt. Im folgenden sind die eingesetzten Katalysatormengen (jeweils 0,38 mmol), die Art des eingesetzten Katalysators und die Adduktausbeuten (jeweils bezogen auf Konjuenester) angegeben:

| Katalysator | Adduktausb. |
|---|---|
| 123,5 mg Rh(NO₃)₃.2H₂O | 17,6 % |
| 90,8 mg (0,19 mmol) [Rh(OAc)₂].2H₂O | 46,6 % |
| 161,1 mg Rh(acac)₃ + 2,0 µl H₂O | 44,1 % |
| 151,4 mg Ru(acac)₃ + 2,0 µl H₂O | 17,3 % |
| 67,4 mg PdCl₂ | 14,8 % |
| 101,1 mg PtCl₂ | 32,3 % |
| 113,5 mg IrCl₃ + 2,0 µl H₂O | 13,6 % |

Es entstanden ausschließlich 1:1-Addukte.

### Beispiel 13.

Ein C₃₆-Guerbetester einer technischen C18:2-Konjuenfettsäure (17,5 g) der in Beispiel 1 angegebenen Zusammensetzung wurde unter den dort angegebenen Reaktionsbedingungen mit Ethylen in Gegenwart von 100 mg RhCl₃.3H₂O und 10 ml Hexan umgesetzt. Die Gesamtausbeute an Addukten betrug 52,4 %, bezogen auf Konjuenester, davon 16,4 % 1:1-, 27,0 % 2:1- und 9,2 % 3:1-Addukte.

## Patentansprüche

1. Olefinisch ungesättigte Addukte von Ethylen an mehrfach ungesättigte Fettsäuren mit 18 bis 22 Kohlenstoffatomen bzw. an Ester derselben mit C₁-C₃₆-Alkanolen in molaren Verhältnissen von Ethylen zu den Fettsäuren bzw. Fettsäureestern im Bereich von 1 : 1 bis 3 : 1, erhältlich durch Umsetzung der Fettsäuren bzw. Fettsäureester mit Ethylen bei erhöhter Temperatur und erhöhtem Druck in Gegenwart von Verbindungen von Übergangsmetallen aus der von Ru, Rh, Pd, Ir und Pt gebildeten Gruppe als Katalysatoren.

2. Olefinisch ungesättigte Addukte nach Anspruch 1, dadurch gekennzeichnet, daß die mehrfach ungesättigten Fettsäuren 2 bis 5, insbesondere 2 bis 3 olefinische Doppelbindungen aufweisen.

3. Olefinisch ungesättigte Addukte nach Anspruch 1 oder 2, dadurch gekennzeichnet, daß man die Addukte bei einem Ethylendruck im Bereich von 5 bis 60 bar und einer Temperatur im Bereich von 50 bis 140°C, gegebenenfalls in Anwesenheit inerter organischer Lösemittel, herstellt.

4. Olefinisch ungesättigte Addukte nach mindestens einem der Ansprüche 1 bis 3, dadurch gekennzeichnet, daß man die Katalysatoren in einer Menge im Bereich von 0,02 bis 2 Mol-%, bezogen auf Fettsäuren bzw. Fettsäureester, verwendet.

5. Olefinisch ungesättigte Addukte nach mindestens einem der Ansprüche 1 bis 4, dadurch gekennzeichnet, daß man als Katalysatoren Rhodiumverbindungen verwendet.

6. Olefinisch ungesättigte Addukte nach mindestens einem der Ansprüche 1 bis 5, dadurch gekennzeichnet, daß man Rhodiumverbindungen aus der von RhCl₃ und RhBr₃ (einschließlich Hydraten derselben) sowie [(C₂H₄)₂RhCl]₂ gebildeten Gruppe als Katalysatoren verwendet.

7. Verfahren zur Herstellung von olefinisch ungesättigten Addukten von Ethylen an mehrfach ungesättigte Fettsäuren mit 18 bis 22 Kohlenstoffatomen bzw. an Ester derselben mit C₁-C₃₆-Alkanolen in molaren Verhältnissen von Ethylen zu den Fettsäuren bzw. Fettsäureestern im Bereich von 1 : 1 bis 3 : 1, gekennzeichnet durch eine Umsetzung der Fettsäuren bzw. Fettsäureester mit Ethylen bei erhöhter Temperatur und erhöhtem Druck in Gegenwart von Verbindungen von Übergangsmetallen aus der von Ru, Rh, Pd, Ir und Pt gebildeten Gruppe als Katalysatoren.

8. Verfahren nach Anspruch 7, dadurch gekennzeichnet, daß man mehrfach ungesättigte Fettsäuren verwendet, die 2 bis 5, insbesondere 2 bis 3 olefinische Doppelbindungen aufweisen.

9. Verfahren nach Anspruch 7 oder 8, dadurch gekennzeichnet, daß man die Addukte bei einem Ethylendruck im Bereich von 5 bis 60 bar und einer Temperatur im Bereich von 50 bis 140°C, gegebenenfalls in Anwesenheit inerter organischer Lösemittel, herstellt.

10. Verfahren nach mindestens einem der Ansprüche 7 bis 9, dadurch gekennzeichnet, daß man die Katalysatoren in einer Menge im Bereich von 0,02 bis 2 Mol-%, bezogen auf Fettsäuren bzw. Fettsäureester, verwendet.

11. Verfahren nach mindestens einem der Ansprüche 7 bis 10, dadurch gekennzeichnet, daß man als Katalysatoren Rhodiumverbindungen verwendet.

12. Verfahren nach mindestens einem der Ansprüche 7 bis 11, dadurch gekennzeichnet, daß man Rhodiumverbindungen aus der von RhCl₃ und RhBr₃ (einschließlich Hydraten derselben) sowie [(C₂H₄)₂RhCl]₂ gebildeten Gruppe als Katalysatoren verwendet.

## Claims

1. Olefinically unsaturated adducts of ethylene with polyunsaturated fatty acids containing 18 to 22 carbon atoms or esters thereof with C₁₋₃₆ alkanols in molar ratios of ethylene to the fatty acids or fatty acid esters of 1:1 to 3:1 obtainable by reaction of the fatty acids or fatty acid esters with ethylene at elevated temperature and elevated pressure in the presence of compounds of the transition metals from the group consisting of Ru, Rh, Pd, Ir and Pt as catalysts.

2. Olefinically unsaturated adducts as claimed in claim 1, characterized in that the polyunsaturated fatty acids contain 2 to 5 and more particularly 2 to 3 olefinic double bonds.

3. Olefinically unsaturated adducts as claimed in claim 1 or 2, characterized in that the adducts are produced under an ethylene pressure of 5 to 60 bar and at a temperature of 50 to 140°C, optionally in the presence of inert organic solvents.

4. Olefinically unsaturated adducts as claimed in at least one of claims 1 to 3, characterized in that the catalysts are used in a quantity of 0.02 to 2 mol-%, based on fatty acids or fatty acid esters.

5. Olefinically unsaturated adducts as claimed in at least one of claims 1 to 4, characterized in that rhodium compounds are used as catalysts.

6. Olefinically unsaturated adducts as claimed in at least one of claims 1 to 5, characterized in that rhodium compounds from the group consisting of RhCl₃ and RhBr₃ (including hydrates thereof) and also [(C₂H₄)₂RhCl]₂ are used as catalysts.

7. A process for the production of olefinically unsaturated adducts of ethylene with polyunsaturated fatty acids containing 18 to 22 carbon atoms or esters thereof with C₁₋₃₆ alkanols in molar ratios of ethylene to the fatty acids or fatty acid esters of 1:1 to 3:1, characterized by reaction of the fatty acids or fatty acid esters with ethylene at elevated temperature and elevated pressure in the presence of compounds of the transition metals from the group consisting of Ru, Rh, Pd, Ir and Pt as catalysts.

8. A process as claimed in claim 7, characterized in that polyunsaturated fatty acids containing 2 to 5 and more particularly 2 to 3 olefinic double bonds are used.

9. A process as claimed in claim 7 or 8, characterized in that the adducts are produced under an ethylene pressure of 5 to 60 bar and at a temperature of 50 to 140°C, optionally in the presence of inert organic solvents.

10. A process as claimed in at least one of claims 7 to 9, characterized in that the catalysts are used in a quantity of 0.02 to 2 mol-%, based on fatty acids or fatty acid esters.

11. A process as claimed in at least one of claims 7 to 10, characterized in that rhodium compounds are used as catalysts.

12. A process as claimed in at least one of claims 7 to 11, characterized in that rhodium compounds from the group consisting of RhCl₃ and RhBr₃ (including hydrates thereof) and also [(C₂H₄)₂RhCl]₂ are used as catalysts.

## Revendications

1. Produits d'addition non saturés oléfiniquement de l'éthylène sur des acides gras plusieurs fois non saturés ayant de 18 à 22 atomes de carbone ou sur un ester de celui-ci avec des alcanols ayant de C₁ à C₃₆ dans des rapports molaires de l'éthylène aux acides gras ou aux esters d'acide gras dans la zone allant de 1:1 à 3:1, que l'on peut produire par mise en réaction des acides gras ou des esters d'acide gras avec l'éthylène, à température élevée et à pression élevée en présence de dérivés de métaux de transition choisis dans le groupe formé de Ru, Rh, Pd, Ir et Pt en tant que catalyseurs.

2. Produits d'addition non saturés oléfiniquement selon la revendication 1, caractérisés en ce que les acides gras non saturés plusieurs fois possèdent de 2 à 5, en particulier de 2 à 3, double liaisons oléfiniques.

3. Produits d'addition non saturés oléfiniquement selon la revendication 1 ou la revendication 2, caractérisés en ce que l'on produit les produits d'addition à une pression d'éthylène dans la zone de 5 à 60 bar et à une température dans la plage de 50 à 140°C, le cas échéant en présence d'un agent dissolvant organique inerte.

4. Produits d'addition non saturés oléfiniquement selon au moins une des revendications 1 à 3, caractérisés en ce que l'on utilise des catalyseurs en quantités dans la zone allant de 0,02 à 2 % mol. rapporté aux acides gras ou à l'ester d'acide gras.

5. Produits d'addition non saturés oléfiniquement selon au moins une des revendications 1 à 4, caractérisés en ce que l'on utilise comme catalyseurs des dérivés du rhodium.

6. Produits d'addition non saturés oléfiniquement selon au moins une des revendications 1 à 5, caractérisés en ce que l'on utilise des dérivés du rhodium choisis dans le groupe formé de RhCl₃ et de RhBr₃ (y compris des hydrates de ceux-ci) ainsi que [(C₂H₄)₂RhCl]₂ en tant que catalyseurs.

7. Procédé d'obtention de produits d'addition non saturés oléfiniquement de l'éthylène sur des acides gras plusieurs fois non saturés ayant de 18 à 22 atomes de carbone, ou un ester de ceux-ci avec des alcanols en C₁ à C₃₆ dans des rapports molaires de l'éthylène aux acides gras ou aux esters d'acide gras dans la zone de 1:1 à 3:1, caractérisé par une réaction des acides gras ou des esters d'acide gras avec l'éthylène à température élevée et à pression élevée en présence de dérivés des métaux de transition choisis dans le groupe formé de Ru, Rh, Pd, Ir et Pt en tant que catalyseurs.

8. Procédé selon la revendication 7, caractérisé en ce que l'on utilise des acides gras plusieurs fois saturés qui possèdent de 2 à 5, en particulier 2 ou 3, double liaisons oléfiniques.

9. Procédé selon la revendication 7 ou la revendication 8, caractérisé en ce que l'on produit les composés d'addition à une pression d'éthylène dans la zone de 5 à 60 bar et à une température dans la plage de 50 à 140°C, le cas échéant en présence d'un agent dissolvant organique inerte.

10. Procédé selon au moins une des revendications 7 à 9, caractérisé en ce que l'on utilise les catalyseurs en quantité dans la zone de 0,02 à 2 % molaire rapporté aux acides gras ou aux esters d'acide gras.

11. Procédé selon au moins une des revendication 7 à 10, caractérisé en ce que l'on utilise comme catalyseurs des dérivés du rhodium.

12. Procédé selon au moins une des revendications 7 à 11, caractérisé en ce que l'on utilise des dérivés du rhodium choisis dans le groupe formé de Cl₃Rh, et de Br₃Rh (y compris les hydrates de ceux-ci) ainsi que de [(C₂H₄)₂RhCl]₂ en tant que catalyseurs.
